(19) Europäisches Patentamt
European Patent Office
Office européen des brevets

(11) Publication number: **0 273 602 A1**

# (12) EUROPEAN PATENT APPLICATION

(21) Application number: 87310603.3

(51) Int. Cl.[4]: **C07D 333/38** , A01N 43/10

(22) Date of filing: 02.12.87

(30) Priority: 05.12.86 US 938220

(43) Date of publication of application: 06.07.88 Bulletin 88/27

(84) Designated Contracting States: AT BE CH DE ES FR GB GR IT LI LU NL SE

(71) Applicant: ELI LILLY AND COMPANY
Lilly Corporate Center
Indianapolis Indiana 46285(US)

(72) Inventor: Abdulla, Riaz Fazal
1310 Bluebird Drive
Greenfield Indiana 46140(US)
Inventor: Morris, Kenneth William
Heel-Cap Cottage Trevilley Lane St. Teath
Bodmin Cornwall PL30 3JS(GB)
Inventor: Williams, James Curtis, Jr.
5153 Brendonshire Court
Indianapolis Indiana 46226(US)

(74) Representative: Tapping, Kenneth George et al
Erl Wood Manor
Windlesham Surrey, GU20 6PH(GB)

(54) **3-Cyano-4-arylthiophenes.**

(57) Herbicidal compounds of formula (I):

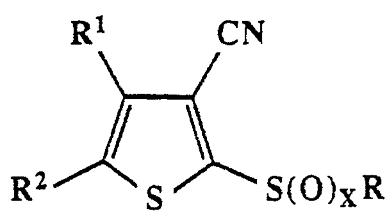

(I)

x is 0-2;
R is $C_1$-$C_3$ primary alkyl;
$R^1$ is phenyl; phenyl mono-substituted with bromo, chloro, fluoro, $C_1$-$C_3$ alkyl, trifluoromethyl, trifluoromethoxy, or $C_1$-$C_3$ alkoxy; or phenyl disubstituted at the 2,4-, 2,6-, or 3,4-positions with bromo, chloro, or fluoro;
$R^2$ is hydrogen or chloro;
provided that
x is 0 and R is methyl when $R^1$ is alkylphenyl or trifluoromethylphenyl; and
R is methyl when $R^1$ is disubstituted phenyl
are particularly useful in rice culture.

EP 0 273 602 A1

# 3-CYANO-4-ARYLTHIOPHENES

This invention belongs to the field of agricultural chemistry, and provides a class of new herbicides and herbicidal methods and compositions making use of the compounds.

U.S. 3,956,315 discloses a genus of 3-cyano-2-ureidothiophene herbicides, which belong to the recognized class of urea herbicides. This invention provides compounds of a different class.

More particularly, this invention provides a series of novel 3-cyanothiophenes of the formula (I)

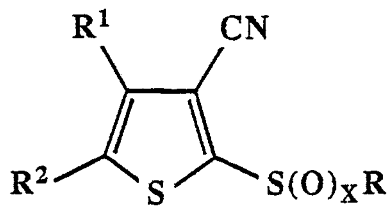

(I)

x is 0-2;
R is $C_1$-$C_3$ primary alkyl;
$R^1$ is phenyl; phenyl mono-substituted with bromo, chloro, fluoro, $C_1$-$C_3$ alkyl, trifluoromethyl, trifluoromethoxy, or $C_1$-$C_3$ alkoxy; or phenyl disubstituted at the 2,4-, 2,6-, or 3,4-positions with bromo, chloro, or fluoro;
$R^2$ is hydrogen or chloro;
provided that
x is 0 and R is methyl when $R^1$ is alkylphenyl or trifluoromethylphenyl; and
R is methyl when $R^1$ is disubstituted phenyl.

The compounds are herbicides which are particularly useful in the culture of rice. Methods of use of the compounds as herbicides and compositions containing the compounds are also provided by the invention.

Throughout this document, all temperatures will be expressed in degrees Celsius. All expressions of concentration, percentage and the like will be expressed in weight measurements unless otherwise stated.

In the above general formula, the term $C_1$-$C_3$ primary alkyl refers to methyl, ethyl and normal propyl. The term $C_1$-$C_3$ alkyl includes those groups, as well as isopropyl, and the term $C_1$-$C_3$ alkoxy refers to $C_1$-$C_3$ alkyl groups linked through an oxygen atom.

Certain classes of the compounds of this invention are preferred. For example, the following limitations set out preferred classes. It will be understood that the limitations below may be combined to constitute further, more limited preferred classes.

A. R is methyl;
B. $R^1$ is phenyl mono-substituted with bromine, chlorine, fluorine, or trifluoromethyl;
C. $R^1$ is 4-mono-substituted-phenyl;
D. $R^1$ is phenyl mono-substituted at the 4-position with bromine, chlorine, fluorine or trifluoromethyl;
E. $R^1$ is phenyl disubstituted with chlorine, bromine or fluorine;
F. $R^2$ is hydrogen;
G. x is 0;
H. x is 1 or 2.

While it is believed that the above general formula and description make the compounds of the invention quite clear, the following exemplary compounds are mentioned to assure understanding.

4-(2-bromophenyl)-5-chloro-3-cyano-2-ethylsulfinylthiophene
3-cyano-4-(4-ethylphenyl)-2-methylthiothiophene
3-cyano-4-(3-fluorophenyl)-2-methylsulfonylthiophene
5-chloro-3-cyano-2-methylthio-4-(3-propylphenyl)thiophene
5-chloro-3-cyano-2-methylthio-4-(2-trifluoromethylphenyl)thiophene
3-cyano-2-propylsulfinyl-4-(4-trifluoromethoxyphenyl)thiophene
3-cyano-2-ethylthio-4-(3-ethoxyphenyl)thiophene
3-cyano-2-propylsulfonyl-4-(2-propoxyphenyl)-thiophene
4-(4-bromo-2-chlorophenyl)-3-cyano-2-methylthiothiophene
4-(2-chloro-6-fluorophenyl)-3-cyano-2-methylsulfinylthiophene
4-(3-bromo-4-fluorophenyl)-3-cyano-5-chloro-2-methylthiothiophene

3-cyano-4-(2,6-difluorophenyl)-2-methylsulfonylthiophene
4-(3,4-dibromophenyl)-3-cyano-2-methylthiothiophene

The compounds of the present invention are readily made by processes not unlike those previously used for making other thiophenes. The most convenient ultimate starting compound is a benzoylacetonitrile, having the desired $R^1$ substitution on the phenyl ring. That compound is reacted with carbon disulfide in the presence of a strong base such as an alkali metal hydride to prepare a salt of the formula

$R^1$-CO- C = C$(SNa)_2$
         |
         CN

The above reaction is conveniently carried out in an inert solvent such as dimethylsulfoxide, at a low or moderate temperature, preferably in the range of 0-25°.

The above intermediate is reacted with an alkyl iodide to prepare the corresponding di(alkylthio) intermediate. The alkyl group of the alkyl iodide corresponds to the group R. The alkylation may conveniently be carried out in the same reaction mixture in which the carbon disulfide reaction was performed, and similarly is carried out at a moderately reduced temperature, or at about ambient temperature. The di(alkylthio) intermediate should be isolated and at least partially purified, as by crystallization from an alkine solvent, before using it in the next step.

Alternatively, the benzoylacetonitrile may be reacted with carbon disulfide and an alkyl iodide wherein the alkyl group is the desired R. The reaction provides the di(alkylthio) intermediate directly. See Example 3 below.

The di(alkylthio) intermediate is then reacted with a lower alkyl thioglycolate and a strong organic base such as triethylamine, to prepare a compound corresponding to the desired thiophene, but having a lower alkyl carboxylate group at the 5-position. The identity of the alkyl group on the thioglycolate is unimportant, since that group will be removed in a later step. The ethyl thioglycolate is preferred, however. The reaction is conveniently carried out in an alkanol, preferably in ethanol, and proceeds best at an elevated temperature. The reflux temperature is most convenient and is preferred, but temperatures in the range of from about the ambient temperature to about 100° may be used as desired, applying pressure to the reaction if it is desired to operate above the normal boiling point.

In the next step, the alkyl ester at the 5-position is converted to the acid, most conveniently, by simple hydrolysis with a strong aqueous base, such as sodium or potassium hydroxode in aqueous ethanol. The carboxy group is then removed by a conventional procedure, such as decarboxylation with copper metal catalyst in an organic base, such as quinoline or picoline.

A 5-chloro substituent is added to the 5-unsubstituted thiophene by reaction with sulfuryl chloride in an inert solvent, preferably a halogenated solvent such as chloroform.

Finally, when an alkylsulfonyl or alkylsulfonyl compound is to be made, the sulfur of the alkylthio group is oxidized to the appropriate degree. Preferably, alkylsulfinyl compounds are prepared by oxidizing with chloroperbenzoic acid, and sulfonyl compounds, by oxidizing with hydrogen peroxide in acetic acid. The oxidations proceed much as similar oxidations of alkylthio groups have been carried out in making analogous compounds.

Accordingly, the invention also provides a process for preparing a compound of formula (I) which comprises

a) decarboxylating a compound of a formula like formula (I) with the exception that $R^2$ is carboxy to produce a compound of formula (I) wherein $R^2$ is hydrogen, or

b) chlorinating a compound of formula (I) wherein $R^2$ is hydrogen to produce a compound of formula (I) wherein $R^2$ is chlorine, or

c) oxidizing a compound of formula (I) wherein x is 0 to produce a compound of formula (I) wherein x is 1, or

d) oxidizing a compound of formula (I) wherein x is 0 or 1 to produce a compound of formula (I) wherein x is 2.

The following synthetic examples are given to assure that the reader can obtain any desired compound of the present invention.

Example 1

3-cyano-2-methylthio-4-(4-trifluoromethylphenyl)thiophene

Two hundred ml of dimethylsulfoxide was added to a flask, and in it was dissolved 20.5 g of 4-trifluoromethylbenzoylacetonitrile. A 7.3 g portion of carbon disulfide was added, and the mixture was cooled to 15°. To it was added, in two portions, 9.2 g of sodium hydride and 100 ml of additional dimethylsulfoxide. The mixture was stirred for 2.5 hours while it warmed to ambient temperature, and then it was cooled to 15° and 27.2 g of methyl iodide was added dropwise. The mixture was stirred overnight and warmed to ambient temperature, and then it was poured into a large amount of water. The aqueous mixture was extracted with 500 ml of ethyl acetate, and the extract was dried over magnesium sulfate, filtered and evaporated under vacuum. The residue was purified by high performance liquid chromatography (HPLC), eluting with 1:1 ethyl acetate:hexane. The product-containing fractions were combined and evaporated under vacuum, and the residue was recrystallized from hexane to obtain 14.6 g of 1-cyano-1-(4-trifluoromethylbenzoyl)-2,2-di(methylthio)ethene, m.p. 60°.

A 13.6 g portion of the above intermediate was dissolved in 200 ml of ethanol, and to it was added 5.8 g of ethyl thioglycolate, with stirring, followed by 4.8 g of triethylamine. The mixture was heated to the reflux temperature, and the heat was turned off as soon as that temperature was reached. The mixture was then cooled, and evaporated under vacuum. The residue was purified by HPLC, eluting with 85:15 hexane:ethyl acetate. The product-containing fractions were combined and evaporated, and the impure product was recrystallized from ethyl acetate to obtain 11.6 g of ethyl 3-cyano-2-methylthio-4-(trifluoromethylphenyl)-5-thiophenecarboxylate, m.p. 105-107°.

A 10.5 g portion of the above ester was added to a stirred mixture of 50 ml of ethanol and 50 ml of 2M sodium hydroxide, and the mixture was stirred for 3-4 hours. It was then filtered through a filter aid pad, and made acidic with concentrated hydrochloric acid. The precipitate was separated by filtration, and was dissolved in ethyl acetate and the solution was washed with water. The solvent was then evaporated under vacuum, and the residue was taken up in acetonitrile.

Some of the above precipitate did not dissolve, but was separated and found to be the desired product of this example, the carboxy group having been removed in the hydrolysis step.

A 3-4 g portion of 5-carboxy-3-cyano-2-methylthio-4-(4-trifluoromethylphenyl)thiophene, as the acetonitrile solution separated above, was combined with 20.2 ml of quinoline and 0.3 g of powdered copper, and the mixture was heated rapidly to 180°. When the evolution of carbon dioxide stopped, the mixture was allowed to cool, and the volatile materials were removed under vacuum. The residue was taken up in dichloromethane and the solution was filtered through a filter aid pad. The filtrate was washed twice with dilute hydrochloric acid, treated with activated charcoal and dried over magnesium sulfate. The solvent was removed under vacuum to obtain 2.1 g of the desired product, m.p. 79-81°.

Theory: C, 52.16; H, 2.69; N, 4.68;
Found: C, 52.18; H, 2.39; N, 4.84.

Example 2

4-(4-chlorophenyl)-3-cyano-2-methylthiophene

The process of Example 1 was followed, in general. A 21.2 g portion of methyl thioglycolate was added to 56.7 g of 1-(4-chlorobenzoyl)-1-cyano-2,2-di-(methylthio)ethene, in 500 ml of ethanol and then 20.0 g of triethylamine was added. The mixture was heated with stirring, and methyl 4-(4-chlorophenyl)-3-cyano-2-methylthio-5-thiophenecarboxylate precipitated as the reaction mixture was cooled. The precipitate was filtered, washed with methanol and recrystallized from acetonitrile to obtain 65.3 g of the intermediate, m.p. 149-151°.

A 44.7 g portion of the above intermediate was saponified by hydrolysis in 400 ml of ethanol and 400 ml of 2M sodium hydroxide. The product was recovered by filtration of the acidified reaction mixture, washed with water and ethanol, and recrystallized from acetonitrile to obtain 30.9 g of the 5-carboxythiophene, m.p. 239-245°.

A 15.5 g portion of the above intermediate was added to 40 ml of quinoline, 1 g of copper bronze was added, and the mixture was heated at 160-180° for 30 minutes. The mixture was then cooled, and the volatile materials were removed under vacuum. The residue was taken up in 100 ml of dichloromethane and filtered, and the filtrate was washed twice with dilute hydrochloric acid, treated with activated charcoal and

dried. The solvent was removed under vacuum, and the residue was recrystallized from cyclohexane to obtain 9.5 g of the desired product, m.p. 100-102°.

Theory: C, 54.23; H, 3.03; N, 5.27;
Found: C, 53.99; H, 2.79; N, 5.33.

Example 3

4-(4-chlorophenyl)-3-cyano-2-ethylthiothiophene

According to the process of the above examples, 71.8 g of 4-chlorobenzoylacetonitrile was reacted with 30.4 g of carbon disulfide and 124.8 g of ethyl iodide in the presence of 80.8 g of triethylamine. The product of that reaction, 68 g, m.p. 62.5-64°, was reacted in methanol with 22.9 g of methyl thioglycolate followed by 21.8 g of triethylamine to obtain 61 g of methyl 4-(4-chlorophenyl)-3-cyano-2-ethylthio-5-thiophenecarboxylate, m.p. 128-132°.

That intermediate was deesterified with sodium hydroxide in aqueous methanol, and the resulting 56 g of the 5-carboxythiophene was decarboxylated in 170 ml of 4-picoline in the presence of 4 g of copper bronze at the reflux temperature. The product was 22.7 g of the desired product, m.p. 44.5-46°.

Theory: C, 55.80; H, 3.60; N, 5.01;
Found: C, 56.06; H, 3.65; N, 5.29.

Example 4

4-(2-chlorophenyl)-3-cyano-2-methylthiothiophene

A 35.7 g portion of methyl 4-(2-chlorophenyl)-3-cyano-2-methylthio-5-thiophenecarboxylate was deesterified with dilute sodium hydroxide in aqueous methanol to obtain 33.3 g of the corresponding 5-carboxy compound, m.p. 208-209.5°. That intermediate was decarboxylated as described in Example 2 in 80 ml of quinoline with 2 g of copper bronze, and the product was isolated according to the general process of Example 2 to obtain 20 g of the desired product, m.p. 92-94°.

Theory: C, 54.23; H, 3.03; N, 5.27;
Found: C, 54.50; H, 2.94; N, 5.11.

Example 5

3-cyano-4-(4-methylphenyl)-2-methylthiothiophene

A 160 g portion of 1-cyano-1-(4-methylbenzoyl)-2,2-di(methylthio)ethene was reacted with 63.6 g of methyl thioglycolate in methanol in the presence of 60.6 g of triethylamine. The intermediate product was 187 g of methyl 3-cyano-4-(4-methylphenyl)-2-methylthio-5-thiophenecarboxylate, which was deesterified with dilute sodium hydroxide to obtain 118.2 g of the 5-carboxy intermediate.

The above intermediate was decarboxylated with quinoline and copper bronze, to obtain 74 g of the desired product, m.p. 73-75°.

Theory: C, 63.64; H, 4.52; N, 5.17;
Found: C, 63.87; H, 4.78; N, 5.55.

Example 6

3-cyano-2-methylthio-4-phenylthiophene

Following the procedures of the examples above, 6.5 g of 5-carboxy-3-cyano-2-methylthio-4-phenylthiophene was prepared and was decarboxylated by heating on a hot plate until the evolution of gas ceased. The dark liquid obtained was cooled until it solidified, and was then taken up in hot toluene. The toluene solution was filtered, and hexane was added to the filtrate to separate a dark oily impurity. The clear filtrate was decanted and evaporated to obtain an oil which solidified upon trituration with hexane. The solid

was separated, washed with hexane and recrystallized from cyclohexane to obtain 1.1 g of the desired product, m.p. 46-47°.

Theory: C, 62.30; H, 3.92; N, 6.05;
Found: C, 62.53: H, 4.01; N, 6.17.

Example 7

3-cyano-2-methylthio-4-(3-trifluoromethylphenyl)thiophene

A 137 g portion of 1-cyano-2,2-di(methylthio)-1-(3-trifluoromethylbenzoyl)ethene was reacted with 46.6 g of methyl thioglycolate and 44.4 g of triethylamine to obtain 107.8 of methyl 3-cyano-2-methylthio-4-(3-trifluoromethylphenyl)-5-thiophenecarboxylate. That intermediate was saponified with sodium hydroxide in aqueous methanol to obtain 97.6 g of the corresponding 5-carboxy intermediate, which was decarboxylated in 250 ml of quinoline with 5 g of copper bronze. The product was isolated as described in Example 2 to obtain 59 g of the desired product, m.p. 73.5-75.5°.

Theory: C, 52.16; H, 2.69; N, 4.68;
Found: C, 52.41; H, 2.75; N, 4.67.

Example 8

4-(3-chlorophenyl)-3-cyano-2-methylthiothiophene

Following the procedures for the examples above, 42 g of 5-carboxy-4-(3-chlorophenyl)-3-cyano-2-methylthiothiophene was prepared, and was decarboxylated with quinoline and copper bronze. The desired product was isolated as described in Example 2 to obtain 25 g, m.p. 50-52°.

Example 9

4-(4-chlorophenyl)-3-cyano-2-propylthiothiophene

Following the procedures of the examples above, 88.5 g of 1-(4-chlorobenzoyl)-1-cyano-2,2-di-(propylthio)ethene was reacted in methanol with 27.6 g of methyl thioglycolate and 26.3 g of triethylamine. The intermediate product, amounting to 34.5 g of the 5-methylcarboxylate, was saponified with sodium hydroxide and aqueous methanol to obtain 26.3 g of the corresponding 5-carboxy intermediate.

A 24 g portion of that intermediate was decarboxylated with 60 ml of quinoline and 2 g of copper bronze and the product was isolated, as described in Example 2, to obtain 14.3 g of the desired product, m.p. 61.5-64°.

Theory: C, 57.23; H, 4.12; N, 4.77;
Found: C, 56.95; H, 3.84; N, 5.00.

Example 10

4-(2,4-dichlorophenyl)-3-cyano-2-methylthiothiophene

Following the processes of the examples above, 135 g of 1-(2,4-dichlorobenzoyl)-1-cyano-2,2-di-(methylthio)ethene was reacted with 49.8 g of methyl thioglycolate and 47.5 g of triethylamine to obtain 18.5 g of the expected 5-thiophenecarboxylate. That intermediate was saponified with sodium hydroxide and aqueous ethanol to obtain 16.7 g of the 5-carboxy intermediate, which was decarboxylated with 75 ml of quinoline and 2 g of copper bronze to obtain 7.5 g of the desired product, m.p. 106-109°.

Theory: C, 48.01; H, 2.35; N, 4.67;
Found: C, 52.36; H, 3.55; N, 5.05.

Example 11

3-cyano-2-methylthio-4-(4-methoxyphenyl)thiophene

Following the procedures of the examples above, 88.8 g of the appropriate 5-thiophenecarboxylate was saponified with sodium hydroxide and aqueous ethanol, to obtain 61g of the 5-carboxy intermediate, which was decarboxylated with 130 ml of quinoline and 3.5 of copper bronze. Isolation of the product according to the process of Example 2 gave 27 g of the desired product, m.p. 75-77°.

Theory: C, 59.74; H, 4.24; N, 5.36;
Found: C, 62.19; H, 4.50; N, 5.05.

Example 12

3-cyano-4-(4-fluorophenyl)-2-methylthiothiophene

Following the general processes in the examples above, 80 g of 1-cyano-1-(4-fluorobenzoyl)-2,2-di-(methylthio)ethene was reacted with 31.8 g of methyl thioglycolate and 30.3 g of triethylamine to prepare the expected methyl 5-thiophenecarboxylate. That intermediate was saponified with sodium hydroxide and aqueous methanol, and was decarboxylated with 30 ml of quinoline and 1 g of copper bronze, substantially as described in Example 2, to obtain 7 g of the desired product, m.p. 50-53°. The product was impure but consisted substantially of the desired compound.

Example 13

5-chloro-4-(4-chlorophenyl)-3-cyano-2-methylthiothiophene

A 10.6 g portion of the compound of Example 2 was dissolved in 100 ml of chloroform, and 5.9 g of sulfuryl chloride was added dropwise with stirring. The mixture was stirred for 30 minutes after the addition was complete, and was then evaporated under vacuum to a solid, which was recrystallized from ethanol to obtain 9.6 g of the desired product, m.p. 124-125°.

Theory: C, 48.01; H, 2.35; N, 4.67;
Found: C, 48.29; H, 2.13; N, 4.54.

Example 14

5-chloro-3-cyano-4-(4-methylphenyl)-2-methylthiothiophene

A 9.8 g portion of the compound of Example 5 was chlorinated as described in Example 13 to obtain 10.6 g of the desired product, m.p. 100-101°.

Theory: C, 55.80; H, 3.60; N, 5.01;
Found: C, 55.63; H, 3.69; N, 4.03.

Example 15

4-(4-chlorophenyl)-3-cyano-2-ethylsulfinylthiophene

Seven g of the compound of Example 3 was dissolved in 100 ml of chloroform and was cooled in an ice bath. To it was added 5.5 g of 85% 3-chloroperbenzoic acid, with stirring. The mixture was stirred for 1 hour, and then more chloroform was added to obtain complete solution. The reaction mixture was then extracted with 2M sodium hydroxide, and the organic layer was dried and was evaporated under vacuum. The residue was recrysallized from acetonitrile to obtain 4.9 g of the.desired product, m.p. 173-175°.

Example 16

4-(2-chlorophenyl)-3-cyano-2-methylsulfinylthiophene

A 6.6 g portion of the compound of Example 4 was oxidized as described in Example 15, and the product was recrystallized from toluene/hexane to obtain 4.5 g of the desired product, m.p. 113-115°.

Theory: C, 51.15; H, 2.86; N, 4.97;
Found: C, 51.44; H, 2.95; N, 4.67.

Example 17

4-(4-chlorophenyl)-3-cyano-2-methylsulfinylthiophene

A 6.6 g portion of the compound of Example 2 was oxidized as described in Example 15 to obtain 5.1 g of the desired product, m.p. 141-144°.

Theory: C, 51.15; H, 2.86; N, 4.97;
Found: C, 51.21; H, 2.98; N, 4.72.

Example 18

3-cyano-4-(4-methylphenyl)-2-methylsulfinylthiophene

A 7.4 g portion of the compound of Example 5 was oxidized as described in Example 15, and the product was recrystallized from toluene, to obtain 3.2 g of the desired product, m.p. 128-130°.

Theory: C, 59.74; H, 4.24; N, 5.36;
Found: C, 60.03; H, 4.44; N, 5.13.

Example 19

4-(3-chlorophenyl)-3-cyano-2-methylsulfinylthiophene

The compound of Example 8 was oxidized as described in Example 15 to obtain 3.5 g of the desired product, m.p. 135-138°.

Theory: C, 51.15; H, 2.86; N, 4.97;
Found: C, 51.25; H, 2.75; N, 5.17.

Example 20

4-(3,4-dichlorophenyl)-3-cyano-2-methylsulfinylthiophene

A 9 g portion of 4-(3,4-dichlorophenyl)-3-cyano-2-methylthiothiophene was made according to the general process of Example 2 above, and was oxidized according to the process of Example 15 to obtain 6.2 g of the desired product, m.p. 141-144° after recrystallization from ethanol.

Theory: C, 45.58; H, 2.23; N, 4.43;
Found: C, 45.72; H, 2.06; N, 4.43.

Example 21

3-cyano-2-methylsulfinyl-4-phenylthiophene

A 6.9 g portion of the compound of Example 6 was oxidized according to the process of Example 15 to obtain 4.3 g of the desired product, m.p. 138-141°.

Theory: C, 58.31; H, 3.66; N, 5.66;
Found: C, 56.70; H, 3.78; N, 5.82.

Example 22

3-cyano-4-(4-methoxyphenyl)-2-methylsulfinylthiophene

A 7.8 g portion of the compound of Example 11 was oxidized as shown in Example 15 to obtain 3.5 g of the desired product, m.p. 119-121°.

Theory: C, 56.30; H, 4.00; N, 5.05;
Found: C, 56.56; H, 4.30; N, 4.76.

Example 23

4-(4-chlorophenyl)-3-cyano-2-ethylsulfonylthiophene

Seven g of the compound of Example 3 was dissolved in 70 ml of acetic acid and 10 ml of hydrogen peroxide solution was added. The mixture was heated to 75°, and the temperature then rose exothermically to 108°. The mixture was allowed to cool and was diluted with a large amount of water. The product precipitated and was recovered by filtration, washed with water and recrystallized from acetonitrile to obtain 6.6 g of the desired product, m.p. 160-163°.

Theory: C, 50.08; H, 3.23; N, 4.49;
Found: C, 50.31; H, 3.42; N, 4.60.

Example 24

4-(4-chlorophenyl)-3-cyano-2-methylsulfonylthiophene

Eight g of the compound of Example 2 was oxidized as described in Example 23 to obtain 6 g of the desired product, m.p. 175-179°.

Theory: C, 48.40; H, 2.71; N, 4.70;
Found: C, 48.65; H, 2.97; N, 4.68.

Example 25

4-(2-chlorophenyl)-3-cyano-2-methylsulfonylthiophene

A 6.6 g portion of the compound of Example 4 was dissolved in acetic acid and oxidized as described in Example 23 to obtain 3.7 g of the desired product, m.p. 159-162°. The product was analyzed by spectral methods and was found to be correctly identified.

Example 26

3-cyano-2-methylsulfonyl-4-phenylthiophene

A 6.9 g portion of the compound of Example 6 was oxidized as described in Example 23, and the product was recrystallized from ethanol to obtain 5.0 g of the desired product, m.p. 163.5-166°.

Theory: C, 54.73; H, 3.44; N, 5.32;
Found: C, 54.62; H, 3.66; N, 5.34.

Example 27

4-(3-chlorophenyl)-3-cyano-2-methylsulfonylthiophene

A portion of the compound of Example 8 was oxidized as described in Example 23 to obtain 5.0 g of the desired product, m.p. 154-157°.
Theory: C, 48.40; H, 2.71; N, 4.70;
Found: C, 48.61; H, 3.00; N, 5.00.

Example 28

5-chloro-4-(4-chlorophenyl)-3-cyano-2-methylsulfonylthiophene

A 5.5 g portion of the compound of Example 14 was oxidized as described in Example 23 to obtain 2.8 g of the desired product, m.p. 195-200°.
Theory: C, 43.38; H, 2.12; N, 4.22;
Found: C, 43.63; H, 1.98; N, 4.44.

Example 29

3-cyano-2-methylsulfonyl-4-(4-trifluoromethylphenyl)thiophene

One g of the compound of Example 1 was oxidized as described in Example 23 to obtain 1.0 g of the desired product, m.p. 184-186°.
Theory: C, 47.13; H, 2.43; N, 4.23;
Found: C, 47.38; H, 2.38; N, 4.45.

Example 30

4-(2,4-dichlorophenyl)-3-cyano-2-methylsulfonylthiophene

Four g of the compound of example 10 was oxidized as described in Example 23 to obtain 3.7 g of the desired product, m.p. 135-135.5°.
Theory: C, 48.56; H, 2.37; N, 4.71;
Found: C, 46.46; H, 2.64; N, 4.14.

Example 31

3-cyano-4-(4-methoxyphenyl)-2-methylsulfonylthiophene

A 7.8 g portion of the compound of Example 11 was oxidized as described in Example 23 to obtain 2.7 g of the desired product, m.p. 123-126°.

Example 32

4-(3,4-dichlorophenyl)-3-cyano-2-methylsulfonylthiophene

Nine g of 4-(3,4-dichlorophenyl)-3-cyano-2-methylthiothiophene was prepared according to the general process of Example 2, and was oxidized according to the process of Example 23, and recrystallized from ethanol, to obtain 4 g of the desired product, m.p. 185-188°.
Theory: C, 43.38; H, 2.12; N, 4.22;
Found: C, 43.19; H, 2.09; N, 4.28.

Testing

Representative compounds of the invention have been tested in standardized test methods designed to evaluate their ability to control weeds. The following reports of such tests illustrate the activity of the compounds.

Test 1

Plant Physiology Screen

The compounds submitted to this test were applied both pre-and postemergence on tomato (Lycopersicon esculentum), large crabgrass (Digitaria sanguinalis), and redroot pigweed (Amaranthus retroflexus) to determine the ability of the compounds to affect growth of these plants in various ways. The compounds were formulated for testing by dissolving a sample of 15 mg of the compound in 0.6 ml of 1:1 acetone-ethanol containing a small amount of a blended anionic-nonionic surfactant, and the solution was diluted with deionized water to a final volume of 6.0 ml. Each formulated compound was applied uniformly over one 66-mm square pot soon after the seeds were planted (preemergence) and to the foliage of the established plants (postemergence) 12 days after the seeds were planted, each pot receiving 3.0 ml of the formulation. The application rate was 15 lb/A.

The treated plants were allowed to grow out in the greenhouse with normal watering and exposure to light, and they were observed 10 to 13 days after treatment, using the following rating scale for plant effect and indications of symptoms.

A. Rating Scale For Plant Effect

1 = no effect
2 = slight effect
3 = moderate effect
4 = severe effect
5 = no emergence or death of plant

B. Symptoms

A = Abscission of leaves
B = Burned
C = Chlorosis
D = Death
E = Epinasty
F = Formative effects other than epinasty
G = Dark green
I = Increased growth
L = Local necrosis
N = No germination
P = Purple pigmentation
R = Reduced germination
S = Stunting
Y = Delayed senescence
Z = Increased branching

Table I

| | | PREEMERGENCE | | | POSTEMERGENCE | | |
|---|---|---|---|---|---|---|---|
| EX. NO. | LB/A | TMTO | LACG | RRPW | TMTO | LACG | RRPW |
| 2 | 15 | 3S | 5N | 5N | 4BS | 5D | 5D |
| 23 | 15 | 3RS | 5N | 5N | 2FS | 4BF | 2B |
| 15 | 15 | 1 | 2S | 3RS | 1 | 1 | 1 |
| 3 | 15 | 1 | 4RS | 4BS | 1 | 4BS | 4SB |
| 24 | 15 | 2B | 3RS | 4RS | 1 | 1 | 1 |
| 16 | 15 | 1 | 2S | 3RS | 1 | 1 | 1 |
| 4 | 15 | 1 | 1 | 3RS | 3SB | 1 | 2BS |
| 25 | 15 | 1 | 1 | 1 | 1 | 1 | 1 |
| 17 | 15 | 5N | 5N | 5N | 5D | 5D | 5D |
| 18 | 15 | 1 | 2S | 2SB | 1 | 1 | 1 |
| 5 | 15 | 1 | 2S | 2S | 1 | 1 | 2FB |
| 26 | 15 | 1 | 4SB | 4BS | 1 | 1 | 1 |
| 6 | 15 | 5N | 5N | 5N | 2SB | 3BS | 3BS |
| 7 | 15 | 2B | 2SB | 3SB | 1 | 1 | 1 |
| 8 | 15 | 1 | 1 | 1 | 1 | 1 | 1 |
| 27 | 15 | 1 | 1 | 1 | 1 | 1 | 1 |
| 19 | 15 | 2S | 2S | 3SB | 1 | 1 | 1 |
| 1 | 15 | 5N | 5N | 5N | 4SB | 5D | 4BS |
| 20 | 15 | 5N | 4RS | 5N | 3SB | 2SB | 2SB |
| 13 | 15 | 5N | 5N | 5N | 4SB | 4BS | 4BS |
| 28 | 15 | 1 | 2S | 5N | 1 | 1 | 1 |
| 14 | 15 | 1 | 1 | 1 | 3FS | 2S | 2F |
| 29 | 15 | 2S | 4SF | 4BS | 1 | 1 | 1 |
| 9 | 15 | 4RS | 4RS | 5N | 4SB | 4BS | 3SB |

Table I

| | | PREEMERGENCE | | | POSTEMERGENCE | | |
|---|---|---|---|---|---|---|---|
| EX. NO. | LB/A | TMTO | LACG | RRPW | TMTO | LACG | RRPW |
| 30 | 15 | 4RS | 4SB | 5N | 4BS | 3BS | 5D |
| 10 | 15 | 3SB | 4SB | 5N | 4BS | 2B | 5D |
| 21 | 15 | 4SB | 4RS | 5D | 1 | 1 | 1 |
| 22 | 15 | 4BS | 4RS | 4SB | 4BS | 2BS | 3BS |
| 31 | 15 | 4BS | 4SR | 4SB | 3BS | 1 | 3BS |
| 11 | 15 | 5N | 5N | 5N | 5D | 2B | 5D |
| 12 | 15 | 5N | 5N | 5N | 5D | 5D | 5D |
| 32 | 15 | 1 | 1 | 4SB | 1 | 1 | 1 |

Test II

Advanced Test

The compounds submitted to this test were applied both pre-and postmergence on species of the following group to determine the ability of the compounds to affect these plants.

A Corn
B Cotton
C Soybean
D Wheat
F Sugarbeets
G Rice
I Tomato
J Barley
L Lambsquarter
N Large Crabgrass
O Mustard
P Pigweed
S Foxtail
U Wild Oats
W Velvetleaf
X Jimsonweed
Z Morningglory

Seeds of the species to be tested were planted in flat pans in a sterile greenhouse soil mixture. Postemergence applications were sprayed over the established plants 8 to 11 days after the seeds were planted and preemergence applications were applied to the soil soon after the seeds were planted.

The compounds were formulated for testing by dissolving a sample in 1:1 acetone-ethanol containing a small amount of a blended anionic-nonionic surfactant. An appropriate amount of the solution was diluted with water to obtain 12.5 ml of dispersion for each flat, containing the correct amount of compound for the desired treatment rate.

The treated plants were allowed to grow out in the greenhouse with normal watering and exposure to light, and the postemergence applications were observed 12 to 14 days after treatment while the preemergence applications were observed 18 to 21 days after treatment using the 1-5 rating scale for plant effect as described earlier.

## Table II
## Preemergence

| RATE LB/A | A | B | C | D | F | G | I | J | L | N | O | P | S | U | W | X | Z |
|---|---|---|---|---|---|---|---|---|---|---|---|---|---|---|---|---|---|
| **Example 2** | | | | | | | | | | | | | | | | | |
| .5 | 1 | 1 | 1 | 1 | 3 | 1 | 1 | 1 | 5 | 4 | 1 | 5 | 5 | 1 | 2 | 2 | 1 |
| .06 | 1 | 1 | 1 | 1 | 1 | 1 | 1 | 1 | 4 | 3 | 1 | 4 | 1 | 1 | 1 | 1 | 1 |
| 2 | 1 | 1 | 1 | 2 | 5 | 1 | 4 | 2 | 5 | 5 | 3 | 5 | 5 | 1 | 3 | 4 | 3 |
| **Example 23** | | | | | | | | | | | | | | | | | |
| .5 | 1 | 1 | 1 | 1 | 3 | 1 | 1 | 1 | 5 | 3 | 1 | 5 | 3 | 1 | 2 | 4 | 1 |
| 4 | 2 | 1 | 2 | 2 | 5 | 2 | 4 | 1 | 5 | 5 | 2 | 5 | 5 | 1 | 5 | 5 | 3 |
| 2 | 2 | 1 | 1 | 1 | 5 | 1 | 3 | 1 | 5 | 5 | 2 | 5 | 5 | 1 | 5 | 5 | 3 |
| **Example 15** | | | | | | | | | | | | | | | | | |
| 8 | | | 1 | | | | 1 | 1 | | 1 | 1 | 1 | 1 | | | | 1 |
| **Example 3** | | | | | | | | | | | | | | | | | |
| .5 | 1 | 1 | 1 | 1 | 1 | 1 | 1 | 1 | 1 | 1 | 1 | 1 | 1 | 1 | 1 | 1 | 1 |
| 4 | 1 | 1 | 1 | 1 | 1 | 1 | 3 | 1 | 5 | 4 | 1 | 5 | 2 | 2 | 3 | 1 | 2 |
| 1 | 1 | 1 | 1 | 1 | 1 | 1 | 1 | 1 | 1 | 3 | 1 | 3 | 1 | 1 | 1 | 1 | 1 |

## Table II

### Preemergence

| RATE LB/A | A | B | C | D | F | G | I | J | L | N | O | P | S | U | W | X | Z |
|---|---|---|---|---|---|---|---|---|---|---|---|---|---|---|---|---|---|
| Example 24 | | | | | | | | | | | | | | | | | |
| .5 | 1 | 1 | 1 | 1 | 1 | 1 | 1 | 1 | 3 | 1 | 1 | 2 | 1 | 1 | 1 | 1 | 1 |
| 8 | | | 1 | | | | 1 | 1 | | 4 | 1 | 3 | 4 | | | | 1 |
| 4 | 1 | 2 | 2 | 1 | 2 | 1 | 2 | 1 | 5 | 3 | 1 | 4 | 1 | 1 | 1 | 1 | 1 |
| Example 16 | | | | | | | | | | | | | | | | | |
| .5 | 1 | 1 | 1 | 1 | 1 | 1 | 1 | 1 | 1 | 1 | 1 | 1 | 1 | 1 | 1 | 1 | 1 |
| 8 | | | 1 | | | | 1 | 1 | | 3 | 1 | 4 | 4 | | | | 1 |
| 4 | 1 | 1 | 1 | 1 | 3 | 1 | 2 | 1 | 5 | 3 | 1 | 5 | 2 | 1 | 4 | 2 | 1 |
| 1 | 1 | 1 | 1 | 1 | 1 | 1 | 1 | 1 | 3 | 1 | 1 | 3 | 1 | 1 | 4 | 1 | 1 |
| Example 4 | | | | | | | | | | | | | | | | | |
| 8 | | | 1 | | | | 1 | 1 | | 3 | 1 | 4 | 1 | | | | 1 |
| Example 25 | | | | | | | | | | | | | | | | | |
| 8 | | | 1 | | | | 2 | 1 | | 2 | 1 | 4 | 3 | | | | 1 |

## Table II

### Preemergence

| RATE LB/A | A | B | C | D | F | G | I | J | L | N | O | P | S | U | W | X | Z |
|---|---|---|---|---|---|---|---|---|---|---|---|---|---|---|---|---|---|
| **Example 17** | | | | | | | | | | | | | | | | | |
| .5 | 1 | 1 | 1 | 1 | 2 | 1 | 2 | 1 | 5 | 4 | 1 | 4 | 3 | 1 | 3 | 1 | 1 |
| 8 | | | 3 | | | | 5 | 2 | | 5 | 3 | 5 | 5 | | | | 5 |
| 2 | 2 | 1 | 2 | 1 | 4 | 1 | 3 | 1 | 5 | 5 | 2 | 5 | 5 | 2 | 3 | 2 | 4 |
| **Example 18** | | | | | | | | | | | | | | | | | |
| 8 | | | 1 | | | | 2 | 1 | | 2 | 1 | 5 | 3 | | | | 1 |
| 4 | 2 | 1 | 1 | 1 | 2 | 1 | 4 | 1 | 4 | 1 | 1 | 2 | 2 | 1 | 2 | 1 | 1 |
| 1 | 1 | 1 | 1 | 1 | 1 | 1 | 1 | 1 | 1 | 1 | 1 | 1 | 1 | 1 | 1 | 1 | 1 |
| **Example 5** | | | | | | | | | | | | | | | | | |
| .5 | 1 | 1 | 1 | 1 | 1 | 1 | 1 | 1 | 1 | 1 | 1 | 1 | 1 | 1 | 1 | 1 | 1 |
| 8 | | | 1 | | | | 1 | 1 | | 2 | 1 | 4 | 4 | | | | 1 |
| 4 | 2 | 1 | 1 | 1 | 2 | 2 | 2 | 1 | 5 | 3 | 1 | 4 | 2 | 1 | 2 | 1 | 2 |
| 1 | 1 | 1 | 1 | 1 | 1 | 1 | 1 | 1 | 4 | 2 | 1 | 3 | 1 | 1 | 1 | 1 | 1 |

## Table II

### Preemergence

| RATE LB/A | A | B | C | D | F | G | I | J | L | N | O | P | S | U | W | X | Z |
|---|---|---|---|---|---|---|---|---|---|---|---|---|---|---|---|---|---|
| **Example 26** | | | | | | | | | | | | | | | | | |
| .5 | 1 | 1 | 1 | 1 | 1 | 1 | 1 | 1 | 1 | 2 | 1 | 1 | 1 | 1 | 1 | 1 | 1 |
| 8 | | | 1 | | | | 3 | 1 | | 4 | 2 | 4 | 4 | | | | 1 |
| 4 | 1 | 1 | 1 | 1 | 4 | 1 | 2 | 1 | 3 | 4 | 1 | 4 | 5 | 1 | 4 | 2 | 1 |
| 2 | 1 | 1 | 1 | 1 | 4 | 1 | 1 | 1 | 1 | 4 | 1 | 4 | 4 | 1 | 4 | 2 | 1 |
| **Example 6** | | | | | | | | | | | | | | | | | |
| 8 | | | 1 | | | | 5 | 1 | | 4 | 1 | 5 | 5 | | | | 3 |
| 4 | 1 | 1 | 1 | 1 | 4 | 1 | 3 | 1 | 5 | 4 | 1 | 4 | 4 | 1 | 4 | 4 | 2 |
| 2 | 1 | 1 | 1 | 1 | 3 | 1 | 2 | 1 | 3 | 3 | 1 | 4 | 2 | 1 | 2 | 3 | 2 |
| 1 | 1 | 1 | 1 | 1 | 1 | 1 | 1 | 1 | 1 | 1 | 1 | 1 | 1 | 1 | 1 | 1 | 1 |
| **Example 7** | | | | | | | | | | | | | | | | | |
| 8 | | | 1 | | | | 1 | 1 | | 4 | 1 | 4 | 2 | | | | 1 |
| 4 | 1 | 1 | 1 | 1 | 1 | 1 | 1 | 1 | 1 | 1 | 1 | 1 | 1 | 1 | 1 | 1 | 1 |

## Table II

### Preemergence

| RATE LB/A | A | B | C | D | F | G | I | J | L | N | O | P | S | U | W | X | Z |
|---|---|---|---|---|---|---|---|---|---|---|---|---|---|---|---|---|---|
| **Example 8** | | | | | | | | | | | | | | | | | |
| 8 | | | 1 | | | | 2 | 1 | | 4 | 1 | 5 | 4 | | | | 2 |
| 4 | 1 | 1 | 1 | 1 | 1 | 1 | 1 | 1 | 1 | 1 | 1 | 4 | 1 | 1 | 1 | 1 | 1 |
| **Example 27** | | | | | | | | | | | | | | | | | |
| 8 | | | 1 | | | | 2 | 1 | | 2 | 1 | 3 | 3 | | | | 1 |
| **Example 19** | | | | | | | | | | | | | | | | | |
| 8 | | | 1 | | | | 3 | 1 | | 4 | 1 | 5 | 4 | | | | 2 |
| 4 | 1 | 2 | 1 | 1 | 3 | 1 | 2 | 1 | 1 | 2 | 1 | 1 | 1 | 2 | 1 | 1 | 1 |
| 2 | 1 | 1 | 1 | 1 | 1 | 1 | 1 | 1 | 1 | 1 | 1 | 1 | 1 | 1 | 1 | 1 | 1 |
| **Example 1** | | | | | | | | | | | | | | | | | |
| .5 | 1 | 1 | 1 | 1 | 1 | 1 | 1 | 1 | 5 | 1 | 1 | 3 | 1 | 1 | 1 | 1 | 1 |
| 8 | | | 1 | | | | 2 | 1 | | 5 | 2 | 5 | 5 | | | | 2 |
| 4 | 1 | 1 | 1 | 1 | 4 | 1 | 3 | 1 | 5 | 5 | 1 | 5 | 4 | 1 | 2 | 1 | 1 |
| 2 | 1 | 1 | 1 | 1 | 3 | 1 | 1 | 1 | 5 | 5 | 1 | 5 | 3 | 1 | 1 | 1 | 1 |

## Table II

### Preemergence

| RATE LB/A | A | B | C | D | F | G | I | J | L | N | O | P | S | U | W | X | Z |
|---|---|---|---|---|---|---|---|---|---|---|---|---|---|---|---|---|---|
| **Example 20** | | | | | | | | | | | | | | | | | |
| .5 | 1 | 1 | 1 | 1 | 1 | 1 | 1 | 1 | 1 | 1 | 1 | 1 | 1 | 1 | 1 | 1 | 1 |
| 8 | | | 1 | | | | 2 | 1 | | 3 | 1 | 5 | 3 | | | | 1 |
| 4 | 2 | 1 | 1 | 1 | 3 | 1 | 3 | 1 | 4 | 4 | 2 | 5 | 4 | 1 | 2 | 1 | 1 |
| 2 | 1 | 1 | 1 | 1 | 2 | 1 | 2 | 1 | 5 | 3 | 2 | 5 | 3 | 1 | 2 | | 1 |
| 1 | 1 | 1 | 1 | 1 | 1 | 1 | 1 | 1 | 4 | 1 | 1 | 4 | 2 | 1 | 1 | 1 | 1 |
| **Example 13** | | | | | | | | | | | | | | | | | |
| 8 | | | 1 | | | | 1 | 1 | | 4 | 1 | 3 | 2 | | | | 1 |
| **Example 28** | | | | | | | | | | | | | | | | | |
| 8 | | | 1 | | | | 1 | 1 | | 1 | 1 | 1 | 1 | | | | 1 |
| **Example 14** | | | | | | | | | | | | | | | | | |
| 8 | | | 1 | | | | 1 | 1 | | 1 | 1 | 1 | 1 | | | | 1 |

## Table II

### Preemergence

| RATE LB/A | A | B | C | D | F | G | I | J | L | N | O | P | S | U | W | X | Z |
|---|---|---|---|---|---|---|---|---|---|---|---|---|---|---|---|---|---|
| **Example 29** | | | | | | | | | | | | | | | | | |
| 8 | | | 1 | | | | 1 | 1 | | 4 | 1 | 4 | 4 | | | | 1 |
| 4 | 1 | 2 | 1 | 1 | 1 | 1 | 1 | 1 | 5 | 3 | 1 | 2 | 4 | 1 | 1 | 1 | 1 |
| 2 | 1 | 1 | 1 | 1 | 1 | 1 | 1 | 1 | 3 | 2 | 1 | 2 | 4 | 1 | 1 | 1 | 1 |
| 1 | 1 | 1 | 1 | 1 | 1 | 1 | 1 | 1 | 1 | 1 | 1 | 1 | 1 | 1 | 1 | 1 | 1 |
| **Example 9** | | | | | | | | | | | | | | | | | |
| 8 | | | 1 | | | | 1 | 1 | | 1 | 1 | 3 | 1 | | | | 1 |
| **Example 30** | | | | | | | | | | | | | | | | | |
| .5 | 1 | 1 | 1 | 1 | 1 | 1 | 1 | 1 | 1 | 1 | 1 | 1 | 1 | 1 | 1 | 1 | 1 |
| 8 | | | 1 | | | | 2 | 1 | | 4 | 2 | 5 | 4 | | | | 1 |
| 2 | 1 | 1 | 1 | 1 | 3 | 1 | 2 | 1 | 2 | 3 | 1 | 4 | 4 | 1 | 2 | 2 | 1 |
| 1 | 1 | 1 | 1 | 1 | 3 | 1 | 1 | 1 | 2 | 2 | 1 | 3 | 3 | 1 | 1 | 1 | 1 |

## Table II

### Preemergence

| RATE LB/A | A | B | C | D | F | G | I | J | L | N | O | P | S | U | W | X | Z |
|---|---|---|---|---|---|---|---|---|---|---|---|---|---|---|---|---|---|
| **Example 10** | | | | | | | | | | | | | | | | | |
| .5 | 1 | 1 | 1 | 1 | 1 | 1 | 1 | 1 | 1 | 1 | 1 | 1 | 1 | 1 | 1 | 1 | 1 |
| 8 | | | 1 | | | | 1 | 1 | | 4 | 2 | 5 | 4 | | | | |
| 4 | 1 | 1 | 1 | 1 | 4 | 1 | 2 | 1 | 4 | 3 | 1 | 5 | 4 | 1 | 1 | 2 | 1 |
| 1 | 1 | 1 | 1 | 1 | 1 | 1 | 1 | 1 | 1 | 1 | 1 | 4 | 3 | 1 | 1 | 1 | 1 |
| **Example 21** | | | | | | | | | | | | | | | | | |
| 8 | | | 1 | | | | 2 | 1 | | 4 | 2 | 5 | 5 | | | | 1 |
| 4 | 1 | 1 | 1 | 1 | 4 | 1 | 2 | 1 | 3 | 3 | 1 | 4 | 4 | 1 | 2 | 2 | 1 |
| 1 | 1 | 1 | 1 | 1 | 1 | 1 | 1 | 1 | 1 | 1 | 1 | 1 | 1 | 1 | 1 | 1 | 1 |
| **Example 22** | | | | | | | | | | | | | | | | | |
| .5 | 1 | 1 | 1 | 1 | 1 | 1 | 1 | 1 | 1 | 1 | 1 | 1 | 1 | 1 | 1 | 1 | 1 |
| 8 | | | 2 | | | | 5 | 1 | | 5 | 4 | 5 | 5 | | | | 3 |
| 4 | 1 | 1 | 1 | 1 | 5 | 1 | 4 | 1 | 5 | 4 | 1 | 5 | 4 | 1 | 4 | 1 | 1 |
| 1 | 1 | 1 | 1 | 1 | 1 | 1 | 1 | 1 | 2 | 1 | 1 | 4 | 1 | 1 | 1 | 1 | 1 |

Table II

Preemergence

| RATE LB/A | A | B | C | D | F | G | I | J | L | N | O | P | S | U | W | X | Z |
|---|---|---|---|---|---|---|---|---|---|---|---|---|---|---|---|---|---|
| Example 31 | | | | | | | | | | | | | | | | | |
| .5 | 1 | 1 | 1 | 1 | 1 | 1 | 1 | 1 | 1 | 1 | 1 | 1 | 1 | 1 | 1 | 1 | 1 |
| 8 | | | 2 | | | | 5 | 1 | | 5 | 4 | 5 | 5 | | | | 1 |
| 4 | 2 | 1 | 1 | 1 | 4 | 1 | 4 | 1 | 3 | 3 | 1 | 5 | 4 | 1 | 2 | 2 | 1 |
| 2 | 2 | 1 | 1 | 1 | 2 | 1 | 2 | 1 | 5 | 3 | 1 | 5 | 2 | 1 | 1 | 1 | 1 |
| 1 | 1 | 1 | 1 | 1 | 1 | 1 | 1 | 1 | 3 | 1 | 1 | 4 | 2 | 1 | 1 | 1 | 1 |
| Example 11 | | | | | | | | | | | | | | | | | |
| .5 | 1 | 1 | 1 | 1 | 1 | 1 | 1 | 1 | 1 | 1 | 1 | 1 | 1 | 1 | 1 | 1 | 1 |
| 8 | | | 1 | | | | 1 | 1 | | 5 | 2 | 5 | 5 | | | | 1 |
| 4 | 1 | 1 | 2 | 1 | 2 | 1 | 3 | 1 | 5 | 4 | 1 | 5 | 5 | 1 | 1 | 1 | 1 |
| 2 | 1 | 1 | 2 | 1 | 1 | 1 | 2 | 1 | 5 | 2 | 1 | 5 | 3 | 1 | 1 | 1 | 1 |
| 1 | 1 | 1 | 2 | 1 | 1 | 1 | 1 | 1 | 4 | 1 | 1 | 2 | 2 | 1 | 1 | 1 | 1 |

Table II

Preemergence

| RATE LB/A | A | B | C | D | F | G | I | J | L | N | O | P | S | U | W | X | Z |
|---|---|---|---|---|---|---|---|---|---|---|---|---|---|---|---|---|---|
| **Example 12** | | | | | | | | | | | | | | | | | |
| .5 | 1 | 1 | 1 | 1 | 3 | 1 | 5 | 1 | 5 | 3 | | 5 | 2 | 1 | 2 | 1 | 1 |
| 8 | | | 2 | | | | 5 | 2 | | 5 | 4 | 5 | 5 | | | | 3 |
| 4 | 1 | 1 | 3 | 1 | 5 | 1 | 5 | 1 | 5 | 5 | 3 | 5 | 5 | 2 | 5 | 5 | 2 |
| 1 | 1 | 1 | 2 | 1 | 4 | 1 | 5 | 1 | 5 | 4 | 2 | 5 | 4 | 1 | 3 | 3 | 1 |
| **Example 32** | | | | | | | | | | | | | | | | | |
| 8 | | | 1 | | | | 2 | 1 | | 1 | 2 | 5 | 1 | | | | 1 |
| 4 | 1 | 1 | 1 | 1 | 2 | 1 | 1 | 1 | 5 | 1 | 1 | 5 | 1 | 1 | 1 | 1 | 1 |
| 2 | 1 | 1 | 1 | 1 | 1 | 1 | 1 | 1 | 4 | 1 | 1 | 5 | 1 | 1 | 1 | 1 | 1 |
| 1 | 1 | 1 | 1 | 1 | 1 | 1 | 1 | 1 | 1 | 1 | 1 | 1 | 1 | 1 | 1 | 1 | 1 |

## Table II

### Postemergence

| RATE LB/A | A | B | C | D | F | G | I | J | L | N | O | P | S | U | W | X | Z |
|---|---|---|---|---|---|---|---|---|---|---|---|---|---|---|---|---|---|
| **Example 2** | | | | | | | | | | | | | | | | | |
| .5 | 2 | 3 | 2 | 1 | 3 | 1 | 3 | 1 | 4 | 1 | 2 | 4 | 2 | 1 | 2 | 4 | 3 |
| 8 | | | 4 | | | | 5 | 2 | | 5 | 4 | 5 | 5 | | | | 5 |
| 2 | 3 | 4 | 3 | 2 | 4 | 1 | 4 | 1 | 5 | 4 | 2 | 5 | 4 | 1 | 4 | 5 | 4 |
| **Example 23** | | | | | | | | | | | | | | | | | |
| .5 | 3 | 2 | 2 | 1 | 0 | 1 | 1 | 1 | 1 | 2 | 1 | 4 | 2 | 1 | 2 | 3 | 2 |
| 8 | | | 3 | | | | 4 | 1 | | 4 | 3 | 5 | 4 | | | | 3 |
| 2 | 3 | 1 | 2 | 1 | 2 | 1 | 1 | 2 | 2 | 1 | 2 | 4 | 4 | 1 | 2 | 5 | 3 |
| **Example 15** | | | | | | | | | | | | | | | | | |
| 8 | | | 1 | | | | 1 | 1 | | 2 | 1 | 2 | 2 | | | | 1 |
| **Example 3** | | | | | | | | | | | | | | | | | |
| .5 | 2 | 2 | 2 | 2 | 2 | 1 | 2 | 1 | 3 | 2 | 2 | 2 | 2 | 2 | 3 | 4 | |
| 8 | | | 3 | | | | 4 | 2 | | 3 | 2 | 4 | 4 | | | | 2 |
| 2 | 3 | 2 | 3 | 2 | 3 | 2 | 3 | 2 | 4 | 2 | 2 | 2 | 3 | 2 | 4 | 4 | 3 |

## Table II

### Postemergence

| RATE LB/A | A | B | C | D | F | G | I | J | L | N | O | P | S | U | W | X | Z |
|---|---|---|---|---|---|---|---|---|---|---|---|---|---|---|---|---|---|
| **Example 24** | | | | | | | | | | | | | | | | | |
| 8 | | | 1 | | | | 1 | 1 | | 1 | 1 | 1 | 1 | | | | 1 |
| **Example 16** | | | | | | | | | | | | | | | | | |
| 8 | | | 1 | | | | 1 | 1 | | 1 | 1 | 1 | 1 | | | | 1 |
| **Example 4** | | | | | | | | | | | | | | | | | |
| 8 | | | 2 | | | | 2 | 1 | | 1 | 1 | 3 | 1 | | | | 2 |
| **Example 25** | | | | | | | | | | | | | | | | | |
| 8 | | | 1 | | | | 1 | 1 | | 1 | 1 | 1 | 1 | | | | 1 |
| **Example 17** | | | | | | | | | | | | | | | | | |
| .5 | 3 | 1 | 3 | 1 | 3 | 1 | 3 | 1 | 5 | 2 | 2 | 3 | 2 | 1 | 2 | 4 | 3 |
| 8 | | | 3 | | | | 4 | 1 | | 4 | 3 | 5 | 2 | | | | |
| 2 | 3 | 3 | 3 | 2 | 3 | 1 | 5 | 1 | 5 | 3 | 3 | 5 | 3 | 2 | 2 | 5 | 3 |

## Table II

### Postemergence

| RATE LB/A | A | B | C | D | F | G | I | J | L | N | O | P | S | U | W | X | Z |
|---|---|---|---|---|---|---|---|---|---|---|---|---|---|---|---|---|---|
| Example 18 | | | | | | | | | | | | | | | | | |
| 8 | | | 1 | | | | 1 | 1 | | 1 | 1 | 1 | 1 | | | | 1 |
| Example 5 | | | | | | | | | | | | | | | | | |
| 8 | | | 2 | | | | 2 | 1 | | 2 | 1 | 2 | 2 | | | | 1 |
| Example 26 | | | | | | | | | | | | | | | | | |
| 8 | | | 1 | | | | 1 | 1 | | 1 | 1 | 1 | 1 | | | | 1 |
| Example 6 | | | | | | | | | | | | | | | | | |
| .5 | 1 | 1 | 1 | 1 | 1 | 1 | 1 | 1 | 1 | 1 | 2 | 1 | 2 | 1 | 1 | 3 | 2 |
| 8 | | | 2 | | | | 3 | 1 | | 2 | 2 | 4 | 4 | | | | 2 |
| 4 | 2 | 1 | 2 | 1 | 1 | 1 | 4 | 1 | 2 | 4 | 2 | 3 | 3 | 1 | 2 | 4 | 2 |
| 2 | 1 | 1 | 2 | 1 | 1 | 2 | 2 | 1 | 1 | 2 | 2 | 2 | 3 | 1 | 3 | 3 | 3 |
| Example 7 | | | | | | | | | | | | | | | | | |
| 8 | | | 1 | | | | 1 | 1 | | 1 | 2 | 3 | 1 | | | | 1 |

Table II

Postemergence

| RATE LB/A | A | B | C | D | F | G | I | J | L | N | O | P | S | U | W | X | Z |
|---|---|---|---|---|---|---|---|---|---|---|---|---|---|---|---|---|---|
| Example 8 | | | | | | | | | | | | | | | | | |
| 8 | | | 2 | | | | 1 | 1 | | 2 | 1 | 2 | 2 | | | | 1 |
| Example 27 | | | | | | | | | | | | | | | | | |
| 8 | | | 1 | | | | 1 | 1 | | 1 | 1 | 1 | 1 | | | | 1 |
| Example 19 | | | | | | | | | | | | | | | | | |
| 8 | | | 2 | | | | 1 | 1 | | 1 | 2 | 3 | 1 | | | | 1 |
| Example 1 | | | | | | | | | | | | | | | | | |
| 8 | | | 2 | | | | 2 | 1 | | 3 | 2 | 4 | 3 | | | | 3 |
| Example 20 | | | | | | | | | | | | | | | | | |
| .5 | 1 | 1 | 2 | 1 | 1 | 1 | 2 | 1 | 3 | 1 | 1 | 3 | 1 | 1 | 1 | | 1 |
| 8 | | | 2 | | | | 4 | 1 | | 2 | 3 | 4 | 2 | | | | |
| 4 | 2 | 2 | 2 | 1 | 3 | 1 | 4 | 1 | 3 | 2 | 2 | 4 | 2 | 1 | 2 | 3 | 2 |
| 2 | 1 | 1 | 2 | 1 | 1 | 1 | 3 | 1 | 3 | 2 | 2 | 3 | 2 | 1 | 1 | | 2 |

Table II

Postemergence

| RATE LB/A | A | B | C | D | F | G | I | J | L | N | O | P | S | U | W | X | Z |
|---|---|---|---|---|---|---|---|---|---|---|---|---|---|---|---|---|---|
| Example 13 | | | | | | | | | | | | | | | | | |
| 8 | | | 1 | | | | 2 | 1 | | 3 | 1 | 4 | 2 | | | | 2 |
| Example 28 | | | | | | | | | | | | | | | | | |
| 8 | | | 1 | | | | 1 | 1 | | 1 | 1 | 1 | 1 | | | | 1 |
| Example 14 | | | | | | | | | | | | | | | | | |
| 8 | | | 2 | | | | 1 | 1 | | 2 | 2 | 3 | 2 | | | | |
| Example 29 | | | | | | | | | | | | | | | | | |
| 8 | | | 1 | | | | 1 | 1 | | 1 | 1 | 1 | 1 | | | | 1 |
| Example 9 | | | | | | | | | | | | | | | | | |
| 8 | | | 2 | | | | 2 | 1 | | 2 | 2 | 4 | 2 | | | | 2 |
| Example 30 | | | | | | | | | | | | | | | | | |
| 8 | | | 2 | | | | 2 | 1 | | 2 | 1 | 3 | 2 | | | | 3 |

## Table II

### Postemergence

| RATE LB/A | A | B | C | D | F | G | I | J | L | N | O | P | S | U | W | X | Z |
|---|---|---|---|---|---|---|---|---|---|---|---|---|---|---|---|---|---|
| **Example 10** | | | | | | | | | | | | | | | | | |
| .5 | 1 | 2 | 2 | 1 | 1 | 1 | 1 | 1 | 1 | 1 | 1 | 3 | 2 | 1 | 1 | | 2 |
| 8 | | | 2 | | | | 3 | 1 | | 2 | 2 | 5 | 2 | | | | 2 |
| 4 | 2 | 3 | 3 | 1 | 3 | 1 | 3 | 1 | 4 | 2 | 2 | 5 | 2 | 1 | 3 | | 3 |
| 1 | 2 | 2 | 2 | 1 | 3 | 1 | 2 | 1 | 3 | 3 | 2 | 5 | 2 | 1 | 1 | 1 | 2 |
| **Example 21** | | | | | | | | | | | | | | | | | |
| 8 | | | 2 | | | | 2 | 1 | | 1 | 1 | 3 | 2 | | | | 2 |
| **Example 22** | | | | | | | | | | | | | | | | | |
| 8 | | | 2 | | | | 3 | 1 | | 2 | 2 | 4 | 2 | | | | 2 |
| **Example 31** | | | | | | | | | | | | | | | | | |
| 8 | | | 3 | | | | 3 | 1 | | 2 | 3 | 4 | 2 | | | | 2 |

## Table II

### Postemergence

| RATE LB/A | A | B | C | D | F | G | I | J | L | N | O | P | S | U | W | X | Z |
|---|---|---|---|---|---|---|---|---|---|---|---|---|---|---|---|---|---|
| **Example 11** | | | | | | | | | | | | | | | | | |
| .5 | 1 | 2 | 2 | 1 | 2 | 1 | 3 | 1 | 1 | 1 | 2 | 2 | 2 | 1 | | 4 | 1 |
| 8 | | | 2 | | | | 5 | 1 | | 2 | 2 | 4 | 2 | | | | 2 |
| 2 | 2 | 2 | 2 | 1 | 3 | 1 | 4 | 1 | 3 | 1 | 2 | 3 | 2 | 1 | | 5 | 2 |
| **Example 12** | | | | | | | | | | | | | | | | | |
| .5 | 2 | 2 | 2 | 2 | 2 | 1 | 4 | 1 | 2 | 1 | 2 | 3 | 2 | 1 | 3 | 4 | 2 |
| 8 | | | 3 | | | | 5 | 2 | | 5 | 4 | 5 | 5 | | | | 5 |
| 2 | 3 | 4 | 3 | 2 | 4 | 3 | 5 | 2 | 3 | 2 | 2 | 5 | 2 | 2 | 4 | 5 | 3 |
| **Example 32** | | | | | | | | | | | | | | | | | |
| 8 | | | 1 | | | | 1 | 1 | | 1 | 1 | 1 | 1 | | | | 1 |

### Test III

### Preemergence Test

A number of compounds were tested, applied to the soil surface or incorporated in the soil, in a preemergence test against another group of plant species. The compounds were formulated essentially as described in Test I above, and were applied to flat pans containing autoclaved greenhouse potting soil. Each treatment was applied at a volume rate of 200 gal/A, and the compound rates were as described in the table below.

Fourteen species of plants were used, as follows.

1 Corn (*Zea mays*)
2 Grain sorghum (*Sorghum vulgare*)
3 Wheat (*Triticum aesitivum*)
4 Morningglory (*Ipomoea sp.*)
5 Foxtail Millet (*Setaria italica*)
6 Velvetleaf (*Abutilon theophrasti*)
7. Pigweed ( *Amaranthus retroflexus*)
8 Soybean (*Glycine max*)
9 Wild Oat (*Avena fatua*)
10 Cotton (*Gossypium hirsutum*)

11 Rice (Oryza sativa)
12 Nightshade (Solanum sp.)
13 Barnyardgrass (Echinochloa crus-galli)
14 Jimsonweed (Datura stramonium )

Seeds of the plants were planted in the treated soil, and the flats were placed in the greenhouse, with appropriate care, to grow out. The effects of the compounds were evaluated 18 to 21 days after treatment, and the results are reported in the following table as percent control.

## Table III

### Surface Applied

| EX. NO. | LB/A | 1 | 2 | 3 | 4 | 5 | 6 | 7 | 8 | 9 | 10 | 11 | 12 | 13 | 14 |
|---|---|---|---|---|---|---|---|---|---|---|---|---|---|---|---|
| 2 | 0.50 | 0 | 20 | 0 | 0 | 100 | 0 | 100 | 0 | 0 | 0 | 0 | 100 | 30 | 30 |
| | 1.00 | 10 | 20 | 0 | 10 | 100 | 20 | 100 | 0 | 0 | 0 | 0 | 100 | 98 | 80 |
| | 2.00 | 30 | 40 | 0 | 20 | 100 | 30 | 100 | 10 | 0 | 0 | 30 | 100 | 98 | 50 |
| 23 | 0.50 | 0 | 10 | 10 | 40 | 100 | 10 | 100 | 0 | 0 | 0 | 0 | 100 | 90 | 20 |
| | 1.00 | 0 | 10 | 20 | 30 | 100 | 60 | 100 | 10 | 0 | 0 | 50 | 100 | 95 | 20 |
| | 2.00 | 20 | 40 | 10 | 30 | 100 | 90 | 100 | 10 | 0 | 0 | 40 | 100 | 100 | 80 |
| 17 | 0.50 | 0 | 0 | 0 | 20 | 95 | 20 | 100 | 0 | 10 | 0 | 0 | 100 | 10 | 0 |
| | 1.00 | 0 | 0 | 10 | 30 | 100 | 70 | 100 | 0 | 10 | 0 | 0 | 100 | 80 | 60 |
| | 2.00 | 20 | 10 | 10 | 30 | 100 | 80 | 100 | 0 | 10 | 0 | 0 | 100 | 95 | 90 |
| 26 | 1.00 | 0 | 0 | 0 | 0 | 10 | 20 | 20 | 0 | 0 | 20 | 0 | 80 | 0 | 0 |
| | 2.00 | 0 | 0 | 0 | 0 | 80 | 50 | 50 | 0 | 0 | 10 | 0 | 100 | 0 | 30 |
| | 4.00 | 20 | 0 | 0 | 30 | 90 | 80 | 50 | 20 | 0 | 10 | 10 | 100 | 0 | 20 |

Table III

Surface Applied

continued

| EX NO. | LB/A | 1 | 2 | 3 | 4 | 5 | 6 | 7 | 8 | 9 | 10 | 11 | 12 | 13 | 14 |
|---|---|---|---|---|---|---|---|---|---|---|---|---|---|---|---|
| 6 | 1.00 | 0 | 0 | 0 | 0 | 0 | 0 | 0 | 0 | 0 | 10 | 0 | | 0 | 0 |
| | 2.00 | 0 | 0 | 0 | 0 | 70 | 30 | 100 | 0 | 10 | 10 | 0 | 80 | 0 | 0 |
| | 4.00 | 0 | 10 | 10 | 0 | 80 | 40 | 100 | 0 | 10 | 10 | 0 | 90 | 0 | 30 |
| 12 | 0.50 | 0 | 0 | 0 | 0 | 40 | 80 | 100 | 0 | 10 | 0 | 10 | 100 | 50 | 80 |
| | 1.00 | 0 | 10 | 0 | 30 | 95 | 80 | 100 | 0 | 10 | 0 | 10 | 100 | 50 | 80 |
| | 2.00 | 20 | 20 | 10 | 60 | 100 | 99 | 100 | 20 | 10 | 0 | 10 | 100 | 95 | 99 |

## Table III

## Pre-Plant Incorporated

| EX. NO. | LB/A | 1 | 2 | 3 | 4 | 5 | 6 | 7 | 8 | 9 | 10 | 11 | 12 | 13 | 14 |
|---|---|---|---|---|---|---|---|---|---|---|---|---|---|---|---|
| 2 | 0.25 | 0 | 0 | 0 | 0 | 0 | 0 | 0 | 0 | 0 | 0 | 0 | 0 | 0 | 0 |
| | 0.50 | 0 | 0 | 0 | 0 | 30 | 0 | 50 | 0 | 0 | 0 | 0 | 98 | 0 | 0 |
| | 1.00 | 0 | 40 | 10 | 30 | 98 | 10 | 98 | 0 | 0 | 0 | 0 | 100 | 0 | 20 |
| 23 | 0.25 | 0 | 0 | 10 | 30 | 0 | 0 | 0 | 0 | 0 | 0 | 0 | 100 | 0 | 0 |
| | 0.50 | 20 | 10 | 10 | 50 | 70 | 0 | 80 | 0 | 0 | 0 | 20 | 100 | 0 | 0 |
| | 1.00 | 40 | 60 | 20 | 70 | 90 | 50 | 100 | 20 | 10 | 0 | 50 | 100 | 60 | 30 |
| 17 | 0.25 | 0 | 0 | 0 | 0 | 0 | 0 | 0 | 0 | 0 | 0 | 0 | 30 | 0 | 0 |
| | 0.50 | 10 | 0 | 10 | 0 | 20 | 0 | 0 | 0 | 10 | 0 | 0 | 98 | 0 | 0 |
| | 1.00 | 30 | 50 | 10 | 40 | 80 | 0 | 60 | 10 | 10 | 0 | 20 | 100 | 20 | 10 |
| 26 | 1.00 | 0 | 0 | 0 | 50 | 0 | 0 | 0 | 0 | 0 | 0 | 0 | | 0 | 0 |
| | 2.00 | 0 | 0 | 0 | 40 | 90 | 90 | 20 | 10 | 0 | 0 | 20 | | 20 | 40 |
| | 4.00 | 20 | 50 | 20 | 40 | 100 | 80 | 50 | 20 | 0 | 0 | 40 | | 60 | 80 |

Table III

Pre-Plant Incorporated

| EX. NO. | LB/A | 1 | 2 | 3 | 4 | 5 | 6 | 7 | 8 | 9 | 10 | 11 | 12 | 13 | 14 |
|---|---|---|---|---|---|---|---|---|---|---|---|---|---|---|---|
| 6 | 1.00 | 0 | 0 | 0 | 0 | 0 | 0 | 40 | 0 | 0 | 0 | 0 | 0 | 0 | 0 |
| | 2.00 | 0 | 0 | 0 | 0 | 0 | 0 | 30 | 0 | 0 | 0 | 0 | 0 | 0 | 0 |
| | 4.00 | 0 | 0 | 0 | 0 | 0 | 0 | 20 | 0 | 0 | 0 | 0 | 0 | 0 | 20 |
| 12 | 0.50 | 0 | 0 | 0 | 30 | 30 | 70 | 100 | 0 | 0 | 0 | 0 | 100 | 0 | 20 |
| | 1.00 | 0 | 50 | 10 | 20 | 40 | 10 | 100 | 0 | 0 | 0 | 0 | 100 | 0 | 98 |
| | 2.00 | 10 | 50 | 10 | 30 | 60 | 98 | 90 | 0 | 20 | 0 | 0 | 100 | 0 | 100 |

Test IV

The compound of Example 2 was tested in a greenhouse preemergence test against 3 crops and 6 weeds. The compound was formulated substantially as described in Test I above, and was applied to the surface of the soil in one-square-meter flats. The volume rate of application was 350 l/ha. The soil was a medium loam containing 4.5% organic matter. Three replicates were applied.

Thirty-three days after treatment and planting, crop injury of the 3 crops was evaluated. Eight days later, crop vigor of the crops and weed control in the 6 weeds were evaluated, with the following results.

The 6 weed species are coded as follows in the table.

A *Alopecurus myosuroides*
B *Avena fatua*
C *Galium aparine*
D *Poa annua*
E *Stellaria media*
F *Veronica persica*

Table IV

CROPS

| APPL'N RATE G/HA | RAPE VIGOR | RAPE INJURY | BARLEY VIGOR | BARLEY INJURY | WHEAT VIGOR | WHEAT INJURY |
|---|---|---|---|---|---|---|
| 250 | 100% | 0 | 100% | 0 | 100% | 0 |
| 500 | 98 | 0 | 100 | 0 | 100 | 0 |
| 750 | 98 | 0 | 100 | 0 | 100 | 0 |
| 1000 | 98 | 0 | 100 | 0 | 99 | 0 |
| 2000 | 95 | 0 | 100 | 0 | 100 | 0 |

Table IV
cont.
WEEDS

| APPL'N RATE G/HA | A | B | C | D | E | F |
|---|---|---|---|---|---|---|
| 250 | 0% | 2% | 0% | 0% | 0% | 0% |
| 500 | 0 | 0 | 0 | 0 | 0 | 0 |
| 750 | 0 | 0 | 0 | 2 | 0 | 3 |
| 1000 | 0 | 0 | 0 | 0 | 0 | 0 |
| 2000 | 0 | 9 | 0 | 3 | 4 | 4 |

Test V

The compound of Example 2 was tested again, in a postemergence test carried out in the greenhouse. The method was essentially like that of Test IV, except that the compound was sprayed over the emerged plants. Barley and wheat were at the 4-leaf stage at application, and the weed species were at various stages from 4 to 8 leaves. The weed species used in this test were as follows.

A *Alopecurus myosuroides*
B *Avena fatua*
C *Galium aparine*
D *Matricaria inodora*
E *Stellaria media*
F *Veronica persica*
G *Viola arvensis*

Crop vigor was rated 17 days after treatment and weed control was rated twice, 24 and 38 days after treatment.

## Table V

### CROP VIGOR

| APPL'N RATE G/HA | WHEAT | BARLEY |
|---|---|---|
| 250 | 94% | 99% |
| 500 | 91 | 98 |
| 1000 | 87 | 99 |
| 2000 | 89 | 97 |

### WEED CONTROL, %

| APPL'N RATE | A | | B | | C | | D | | E | | F | | G | |
|---|---|---|---|---|---|---|---|---|---|---|---|---|---|---|
| G/HA | 24 | 38 | 24 | 38 | 24 | 38 | 24 | 38 | 24 | 38 | 24 | 38 | 24 | 38 |
| 250 | 0 | 0 | 0 | 0 | 1 | 0 | 1 | 0 | 0 | 0 | 7 | 0 | 0 | 0 |
| 500 | 0 | 0 | 0 | 0 | 3 | 0 | 0 | 0 | 0 | 0 | 45 | 22 | 2 | 0 |
| 1000 | 0 | 0 | 0 | 0 | 6 | 2 | 0 | 0 | 0 | 0 | 74 | 68 | 4 | 0 |
| 2000 | 0 | 0 | 0 | 0 | 9 | 3 | 2 | 0 | 3 | 1 | 91 | 97 | 6 | 2 |

Test VI

The compound of Example 2 was tested in field plots in the midwestern United States against a number of weeds and crops. In this test, the compound was formulated as 1 lb/gal emulsifiable concentrate, and was applied at the volume rate of 25 gal/A. Appropriate amounts of the formulation were dispersed in water to make up the treatments to be applied. All treatments were applied to the surface of the soil, which was a medium silt loam, and the crops and weeds were seeded immediately after application.

The 6 crops were observed for crop injury 19 days and 28 days after treatment, and the weeds were observed for weed control 22 days after treatment. All ratings were on the 0-10 scale, where 0 indicates no control of the weed or no crop injury, and 10 indicates death of the plants or no emergence. Intermediate numbers indicate intermediate degrees of weed control or crop injury.

The species in the test were as follows.

A Soybean
B Cotton, Upland
C Barley
D Rice
E Wheat, Common
F Corn, Field
G Velvetleaf
H Pigweed, Redroot
I Mustard, Black

J Lambsquarters, Common
K Jimsonweed
L Morningglory
M Setaria Spp.
N Foxtail, Green
O Cocklebur, Common

Table VI

WEED CONTROL

| RATE LB/A | L | H | K | M | J | I | G | N | O |
|---|---|---|---|---|---|---|---|---|---|
| 0.5 | 0 | 0 | 0 | 0 | 0 | 0 | 0 | 3 | 0 |
| 1 | 0 | 5 | 0 | 0 | 5 | 0 | 3 | 6 | 0 |
| 2 | 0 | 7 | 4 | 9 | 9 | 4 | 3 | 9 | 0 |
| 4 | 4 | 10 | 9 | 9.8 | 9.5 | 7 | 4 | 9.8 | 0 |

CROP INJURY

| RATE | F | | E | | D | | B | | C | | A | |
|---|---|---|---|---|---|---|---|---|---|---|---|---|
| LB/A | 19 | 28 | 19 | 28 | 19 | 28 | 19 | 28 | 19 | 28 | 19 | 28 |
| 0.5 | 0 | 0 | 0 | 0 | 0 | 0 | 0 | 0 | 0 | 0 | 0 | 0 |
| 1 | 0 | 0 | 0 | 0 | 0 | 0 | 0 | 0 | 0 | 0 | 0 | 0 |
| 2 | 0 | 0 | 0 | 0 | 0 | 0 | 0 | 0 | 1 | 0 | 0 | 0 |
| 4 | 2 | 0 | 0 | 0 | 0 | 0 | 0 | 2 | 0 | 0 | 2 | 0 |

Test VII

An experiment in submerged, transplanted rice was carried out with the compound of Example 2. The 16-day-old rice plants were transplanted into large pots, and were covered with water. Four weed species were sowed in the pots at the time of transplanting, and the compound was applied below the surface of the water 4 days after transplanting. Crop injury was observed 7 days after treatment, and crop vigor was observed 14 days after treatment. The weed control produced by the compound was observed 3 times, 14, 21 and 28 days after treatment, and was recorded in terms of percent control of the weed, compared to untreated controls. The weeds used in the test were as follows.

A Broadleaves, Annual
B *Cyperus difformis*
C *Echinochloaspp.*
D *Monochoria vaginalis*

Table VII

| APPL'N RATE G/HA | RICE INJURY | RICE VIGOR |
|---|---|---|
| 1000 | 0 | 100% |
| 1500 | 0 | 100 |
| 2000 | 0 | 100 |

WEED CONTROL, %

| APPL'N RATE | A | | | B | | | C | | | D | | |
|---|---|---|---|---|---|---|---|---|---|---|---|---|
| G/HA | 14 | 21 | 28 | 14 | 21 | 28 | 14 | 21 | 28 | 14 | 21 | 28 |
| 1000 | 100 | 67 | 100 | 100 | 100 | 100 | 100 | 93 | 100 | 17 | 93 | 100 |
| 1500 | 83 | 60 | 100 | 100 | 67 | 100 | 90 | 63 | 100 | 67 | 60 | 100 |
| 2000 | 100 | 67 | 100 | 100 | 100 | 100 | 93 | 93 | 100 | 50 | 93 | 100 |

The herbicidal activity of the compounds of this invention is illustrated by the above examples. The test results particularly show the safety of the compounds in rice, and the effectiveness of them against weeds growing in rice culture. However, plant scientists will recognize that the exemplified activity of the compounds shows that they are broadly effective against unwanted herbaceous plants, which will be referred to as weeds, when used at appropriate application rates and applied in the appropriate manner.

As the above test results demonstrate, the compounds are used to reduce the vigor of weeds by contacting them with an herbicidally-effective amount of one of the compounds. The term "reduce the vigor of" is used to refer to both killing and injuring the weed which is contacted with a compound. In some instances, as is clear from the test results, the whole population of the contacted weed is killed. In other instances, part of the weeds are killed and part of them are injured, and in still other instances, none of the weeds is killed but they are merely injured by application of the compound. It will be understood that reducing the vigor of the weed population by injuring part of them is beneficial, even though part of the population survives application of the compound. The weeds, the vigor of which has been reduced, are unusually susceptible to the stresses which normally afflict plants, such as disease, drought, lack of nutrients and so forth.

Thus, the treated weeds are likely to expire due to stress of the environment, even though they survive application of the compound. Further, if the treated weeds are growing in cropland, the crop, as it grows normally, tends to shade out the treated weeds of reduced vigor. Therefore, the crop has a great advantage over the treated weeds in the competition for nutrients and sunlight. Still further, when the treated weeds are growing in fallow land, or industrial property which is desired to be bare, the reduction in their vigor necessarily tends to minimize the treated weeds' consumption of water and nutrients, and also minimizes the fire hazard and nuisance which the weeds present.

The compounds are herbicidally effective when applied both preemergence and postemergence. Thus, they can be used both by direct contact of the compounds with emerged weeds, and by applying the compounds to the soil, where they come into contact with germinating and emerging weeds. Preemergence application of the compounds, wherein the germinating and emerging weeds are contacted with the compound through soil application, is preferred.

Accordingly, an important embodiment of this invention is a method of reducing the vigor of weeds which comprises contacting the weeds with an herbicidally-effective amount of a compound of the invention.

The term herbicidally-effective amount refers to an amount which will reduce the vigor of the treated weed. In the context of this invention, weed seeds, which are contacted with the compounds by application of the compounds to the soil, are regarded as weeds.

Amounts of herbicides are measured in terms of the weight of herbicide applied per unit area, usually called the application rate. The best application rate of a given compound of the invention for the control of a given weed varies, of course, depending upon the climate, soil texture, water and organic matter contents of the soil and other factors known to those skilled in plant science. It will be found, however, that the optimum application rate is usually in the range from about 0.25 to about 10 kg/ha, preferably about 0.5-5 kg/ha.

It is not implied, of course, that all compounds of the invention are effective against all weeds at all rates. Some compounds are most effective against some types of weeds, other compounds are more effective against other types. All of the compounds, however, are effective against at least some weeds. It is within the ordinary skill of a plant scientist to ascertain the weeds which are most advantageously controlled with the various compounds, and the best application rate for the particular use.

Use of the compounds in rice culture is particularly preferred. Rice, of course, is very frequently grown in paddy culture; that is, the plants are transplanted and the field is flooded and kept flooded through the major part of the plants' growing life. Compounds of the present invention may be applied to the field, before the rice is transplanted and the field is flooded, or may be applied to the water after the field is flooded. In either event, it is preferred to apply the compound at the time of transplanting. The usual types of compositions are used for applications in rice culture; it is often found that granular compositions are particularly useful for the purpose.

The compounds are applied to the soil, to paddy water, or to emerged weeds in the manners usual in agriculture. It is best to apply the compounds in the form of the herbicidal compositions which are important embodiments of the present invention. The compositions comprise suitable mixtures of the compounds with agriculturally-acceptable diluents. They may be applied to the soil in the form of either water-dispersed or granular compositions, the preparation of which will be discussed below. Usually, water-dispersed compositions will be used for the application of the compounds to emerged weeds. The compositions are applied with any of the many types of sprayers and granular applicators which are in wide use for the distribution of agricultural chemicals over soil or standing vegetation. In general, the compositions are formulated in the manners usual in agricultural chemistry.

Very often, the compounds are formulated as concentrated compositions which are applied either to the soil or the foliage in the form of water dispersions or emulsions containing in the range of from about 0.1 percent to about 5 percent of the compound. Water-dispersible or emulsifiable compositions are either solids usually known as wettable powders, or liquids usually known as emulsifiable concentrates. Wettable powders comprise an intimate, finely-divided mixture of the compound, an inert carrier, and surfactants. The concentration of the compound is usually from about 10 percent to about 90 percent. The inert carrier is usually chosen from among the attapulgite clays, the montmorillonite clays, the kaolin clays, the diatomaceous earths and the purified silicates. Effective surfactants, comprising from about 0.5 percent to about 10 percent of the wettable powder, are found among the sulfonated lignins, the condensed naphthalenesulfonates, the naphthalenesulfonates, the alkylbenzensulfonates, the alkyl sulfates and nonionic surfactants such as ethylene oxide adducts of phenol.

Typical emulsifiable concentrates of the new compounds comprise a convenient concentration of the compound, such as from about 100 to about 500 g per liter of liquid, dissolved in an inert carrier which is a mixture of water-immiscible solvent and emulsifiers. Useful organic solvents include the aromatics, especially the xylenes, and the petroleum fractions, especially the high-boiling napthalenic and olefinic portions of petroleum. Many other organic solvents may also be used such as the terpenic solvents, and the complex alcohols such as 2-ethoxyethanol. Suitable emulsifiers for emulsifiable concentrates are chosen from the same types of surfactants used for wettable powders.

When a compound is to be applied to the soil, as for a preemergence application of the compound, it is convenient to use a granular formulation. Such a formulation typically comprises the compound dispersed on a granular inert carrier such as coarsely ground clay. The particle size of granules usually ranges from about 0.1 to about 3 mm. The usual formulation process for granules comprises dissolving the solution to the carrier in an appropriate solids mixer. Somewhat less economically, the compound may be dispersed in a dough composed of damp clay or other inert carrier, which is then dried and coarsely ground to produce the desired granular product.

A particularly convenient formulation for compounds of the present invention is exemplified by the following emulsifiable concentrate.

| | |
|---|---|
| Compound of Example 2 | 10% by weight |
| Toximul H (anionic/nonionic surfactant blend) | 10% |
| Propylene Glycol Monomethyl Ether | 20% |
| Xylene | 60% |

## Claims

1. A compound of the formula

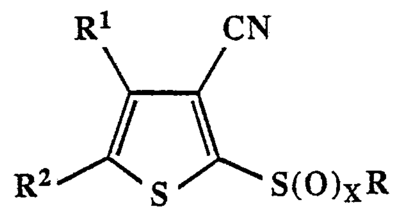

(I)

x is 0-2;
R is $C_1$-$C_3$ primary alkyl;
$R^1$ is phenyl; phenyl mono-substituted with bromo, chloro, fluoro, $C_1$-$C_3$ alkyl, trifluoromethyl, trifluoromethoxy, or $C_1$-$C_3$ alkoxy; or phenyl distributed at the 2,4-2,6-, or 3,4-positions with bromo, chloro, or fluoro;
$R^2$ is hydrogen or chloro;
provided that
x is 0 and R is methyl when $R^1$ is alkylphenyl or trifluoromethylphenyl; and
R is methyl when $R^1$ is disubstituted phenyl.

2. A compound of Claim 1 wherein R is methyl.

3. A compound of Claim 1 or 2 wherein $R^2$ is hydrogen.

4. A compound of Claim 2 wherein x is 0.

5. A compound of Claim 2 or 4 wherein $R^1$ is mono-(bromo, chloro, fluoro or trifluoromethyl)phenyl.

6. The compound of Claim 1 which is 4-(4-chlorophenyl)-3-cyano-2-methylthiothiophene.

7. The compound of Claim 1 which is 4-(4-chlorophenyl)-3-cyano-2-methylsulfonylthiophene.

8. The compound of Claim 1 which is 4-(4-chlorophenyl)-3-cyano-2-methylsulfinylthiophene.

9. An agricultural composition comprising a compound of any one of Claims 1-8 and an agriculturally-acceptable diluent.

10. A method of reducing the vigor of weeds which comprises contacting the weeds with an herbicidally-effective amount of a compound of any one of Claims 1-8.

11. A process for preparing a compound of formula (I), as defined in Claim 1, which comprises:

a) decarboxylating a compound of a formula like formula (I) with the exception that $R^2$ is carboxy to produce a compound of formula (I) wherein $R^2$ is hydrogen, or

b) chlorinating a compound of formula (I) wherein $R^2$ is hydrogen to produce a compound of formula (I) wherein $R^2$ is chlorine, or

c) oxidizing a compound of formula (I) wherein X is 0 to produce a compound of formula (I) wherein X is 1, or

d) oxidizing a compound of formula (I) wherein X is 0 or 1 to produce a compound of formula (I) wherein X is 2.

Claims for the following contracting state: ES

1. An agricultural composition comprising as active ingredient a compound of formula (I)

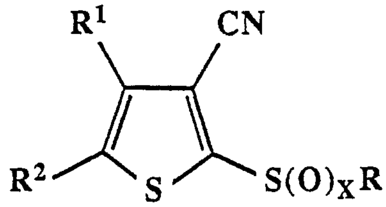

(I)

x is 0-2;
R is $C_1$-$C_3$ primary alkyl;
$R^1$ is phenyl; phenyl mono-substituted with bromo, chloro, fluoro, $C_1$-$C_3$ alkyl, trifluoromethyl, trifluoromethoxy, or $C_1$-$C_3$ alkoxy; or phenyl disubstituted at the 2,4-, 2,6-, or 3,4-positions with bromo, chloro, or fluoro;
$R^2$ is hydrogen or chloro;
provided that
x is 0 and R is methyl when $R^1$ is alkylphenyl or trifluoromethylphenyl; and
R is methyl when $R^1$ is disubstituted phenyl. in combination with an agriculturally-acceptable diluent.

2. A composition of claim 1 which is an emulsifiable concentrate comprising a compound of formula (I) dissolved in an inert carrier which is a mixture of water-imiscible solvent and emulsifier.

3. A composition of claim 2 containing 100 to 500 g/L of the compound of formula (I).

4. A composition of any one of claims 1-3 wherein the compound of formula (I) is one wherein R is methyl.

5. A composition of claim 4 wherein the compound of formula (I) is one wherein $R^2$ is hydrogen.

6. A composition of claim 4 wherein the compound of formula (I) is one wherein x is 0.

7. A composition of claim 4 wherein the compound of formula (I) is one wherein $R^1$ is mono-(bromo, chloro, fluoro, or trifluoromethyl)phenyl.

8. A composition of claim 4 wherein the compound of formula (I) is selected from:
4-(4-chlorophenyl)-3-cyano-2-methylthiothiophene.
4-(4-chlorophenyl)-3-cyano-2-methylsulfonylthiophene.
4-(4-chlorophenyl)-3-cyano-2-methylsulfinylthiophene.

9. A process for preparing a compound of formula (I), as defined in Claim 1, which comprises:

a) decarboxylating a compound of a formula like formula (I) with the exception that $R^2$ is hydrogen, or

b) chlorinating a compound of formula (I) wherein $R^2$ is hydrogen to produce a compound of formula (I) wherein $R^2$ is chlorine, or

c) oxidizing a compound of formula (I) wherein X is 0 to produce a compound of formula (I) wherein X is 1, or

d) oxidizing a compound of formula (I) wherein X is 0 or 1 to produce a compound of formula (I) wherein X is 2.

European Patent Office

# EUROPEAN SEARCH REPORT

Application Number

EP 87 31 0603

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| P,A | EP-A-0 234 622 (DUPHAR INTERNATIONAL RESEARCH B.V.)<br>* claims 4, 5, 13 * | 1,9 | C 07 D 333/38<br>A 01 N 43/10 |
| A | EP-A-0 031 173 (DUPHAR INTERNATIONAL RESEARCH B.V.)<br>* claims 1-3, 7 * | 1,9,11 | |
| A | DE-A-2 510 936 (CHEVRON RESEARCH CO.)<br>* claims 1, 9, 10 * & US - A - 3 956 315 (Cat. D,A) | 1,9,10 | |
| A | GB-A-1 334 015 (SHELL INTERNATIONALE RESEARCH MAATSCHAPPIJ N.V.)<br>* claims 1, 10, 11; page 5, table I, compounds IC, ID, IE * | 1,9,10 | |
| P,A | EP-A-0 207 609 (E.I. DU PONT DE NEMOURS AND CO.)<br>* page 41, general formula I; table I, page 54, lines 15-20; abstract * | 1,9,10 | TECHNICAL FIELDS SEARCHED (Int. Cl.4) |
| A | EP-A-0 024 042 (E.I. DU PONT DE NEMOURS AND CO.)<br>* claims 1, 11-13 * | 1,11 | C 07 D 333/00 |

The present search report has been drawn up for all claims

| Place of search | Date of completion of the search | Examiner |
|---|---|---|
| BERLIN | 26-02-1988 | HASS C V F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)